Europäisches Patentamt

(19) European Patent Office

Office européen des brevets

(11) **EP 0 595 949 B1**

(12) **FASCICULE DE BREVET EUROPEEN**

(45) Date de publication et mention
de la délivrance du brevet:
**19.06.1996 Bulletin 1996/25**

(21) Numéro de dépôt: 92916072.9

(22) Date de dépôt: 16.07.1992

(51) Int. Cl.$^6$: **A61K 7/48**, A61K 7/06,
A61K 7/13

(86) Numéro de dépôt international:
**PCT/FR92/00689**

(87) Numéro de publication internationale:
**WO 93/01795 (04.02.1993 Gazette 1993/04)**

(54) **COMPOSITIONS POUR LA PIGMENTATION DE LA PEAU OU DES CHEVEUX CONTENANT UN EXTRAIT DE MARRUBIUM VULGARE**

EXTRAKT AUS MARRUBIUM VULGARE ENTHALTENDE ZUSAMMENSETZUNGEN ZUR PIGMENTIERUNG DER HAUT ODER DER HAARE

COMPOSITIONS FOR THE PIGMENTATION OF THE SKIN OR OF THE HAIR CONTAINING AN EXTRACT OF MARRUBIUM VULGARE

(84) Etats contractants désignés:
**DE ES FR GB IT**

(30) Priorité: **19.07.1991 FR 9109198**

(43) Date de publication de la demande:
**11.05.1994 Bulletin 1994/19**

(73) Titulaire: **LVMH RECHERCHE
F-92752 Nanterre (FR)**

(72) Inventeurs:
  • **MEYBECK, Alain
    Les Poissons
    F-92400 Courbevoie (FR)**
  • **BONTE, Frédéric
    F-92400 Courbevoie (FR)**
  • **DUMAS, Marc
    F-92700 Colombes (FR)**

(74) Mandataire: **Portal, Gérard et al
    Cabinet Beau de Loménie
    158, rue de l'Université
    75340 Paris Cédex 07 (FR)**

(56) Documents cités:
    **GB-A- 1 098 065**

    • **Manufacturing Chemist and Aerosol News, vol. 53, no. 7, juillet 1982, (Londres, GB), "Extracts of plants", voir Horehound**
    • **STN Serveur de Bases de Donnees, Karlsruhe DE, Fichier Chemical Abstracts, vol. 86, no. 1, résumé no. 2355u (Colombus, Ohio, US), M.O. Karryev et al.: "Some therapeutic properties and phytochemistry of common horehound", & IZV. AKAD. NAUK TURKM. SSR, SER. BIOL. NAUK, 1976 (3), 86-8, voir le résumé**
    • **STN Serveur de Bases de Donnees, Karlsruhe DE, Fichier Chemical Abstracts, vol. 91, no. 15, résumé no. 120333m, (Columbus, Ohio, US), G.I. NILOV et al.: "Amino acid composition and antimicrobial properties of vegetative material infusions of spice plants", & DEPOSITED DOC., VINITI 320-78, 1978, 15 PP, voir le résumé**

**Description**

La présente invention concerne essentiellement une composition cosmétique ou pharmaceutique, notamment dermatologique, contenant un extrait de Marrubium vulgare, destinée en particulier à favoriser la pigmentation de la peau ou des cheveux, et son procédé de fabrication.

La présente invention concerne aussi l'utilisation d'un extrait de Marrubium vulgare pour la préparation d'une telle composition cosmétique ou pharmaceutique, en particulier destinée à traiter les troubles de la pigmentation.

Les différentes espèces du genre Marrubium vulgare font partie de la famille des Labiées. Le Marrubium vulgare encore dénommé Marrube blanc se rencontre communément dans les lieux incultes surtout dans les régions méditerranéennes ainsi qu'en Asie occidentale et en Afrique du Nord.

La tradition attribue aux feuilles et aux sommités fleuries de cette plante des propriétés expectorantes et fluidifiantes des sécrétions bronchiques. Elle aurait en outre un effet sur les arythmies extrasystoliques (voir notamment le Précis de Matière Médicale par R. R. PARIS et Mme H. MOYSE, tome III, publié chez MASSON et Compagnie, Editeurs, 1971, page 293).

Le document GB-1 098 065 décrit une composition pharmaceutique administrée par voie orale ayant notamment une action antispasmodique, diminuant l'effet hypertenseur de l'adrénaline et ayant une action anti-sérotonine, avec un excipient adapté à l'administration orale pour réaliser des comprimés ou des cachets (page 2, lignes 42 à 48).

La présente invention est basée sur la découverte que les extraits de Marrubium vulgare, provenant en particulier des parties aériennes et spécialement des tiges et des feuilles, présentent des propriétés biologiques intéressantes utilisables dans les domaines cosmétique et pharmaceutique. Il a notamment été observé par les inventeurs que ces extraits possédaient de façon inattendue une activité stimulante de la mélanogénèse, au niveau des mélanocytes présents dans la peau ou les follicules pileux, notamment ceux du cuir chevelu, et permettaient ainsi de favoriser la pigmentation de la peau ou des cheveux, ainsi que de réaliser un traitement des désordres de la pigmentation de la peau et des cheveux en favorisant plus particulièrement la biosynthèse de mélanine. De très bons résultats dans ce domaine ont été obtenus avec des extraits des parties aériennes et en particulier des tiges et des feuilles de Marrubium vulgare.

La présente invention a donc pour but de résoudre le nouveau problème technique consistant en la fourniture d'une nouvelle composition cosmétique ou pharmaceutique, notamment dermatologique, destinée en particulier à favoriser la pigmentation de la peau, à prévenir ou ralentir l'apparition des cheveux blancs.

La présente invention a encore pour but de résoudre le nouveau problème technique consistant en la préparation d'une nouvelle formulation d'une composition cosmétique ou pharmaceutique présentant une bonne activité de stimulation de la mélanogénèse au niveau des mélanocytes présents dans la peau ou dans les follicules pileux, notamment ceux du cuir chevelu.

La présente invention a encore pour but de fournir une solution au nouveau problème technique consistant en la fourniture d'un extrait de plante particulièrement aisé à obtenir, qui présente en lui-même une bonne activité stimulante de la mélanogénèse, au niveau des mélanocytes présents dans la peau ou les follicules pileux, sans avoir à réaliser l'isolation d'une substance active quelconque, des procédés d'isolation étant en général longs et coûteux.

Tous ces nouveaux problèmes techniques sont résolus pour la première fois par la présente invention d'une manière satisfaisante, utilisable à l'échelle industrielle.

Ainsi, selon un premier aspect, la présente invention concerne une composition cosmétique ou pharmaceutique, notamment dermatologique, sous une forme destinée à l'administration superficielle sur la peau ou le cuir chevelu, destinée en particulier à favoriser la pigmentation de la peau, à prévenir ou ralentir l'apparition des cheveux blancs, caractérisée en ce qu'elle comprend à titre d'ingrédient actif une quantité cosmétiquement ou pharmaceutiquement efficace d'un extrait de Marrubium vulgare, éventuellement dans un excipient cosmétiquement ou pharmaceutiquement acceptable.

Dans la présente description et les revendications, on entend par "quantité cosmétiquement ou pharmaceutiquement efficace" une quantité au moins égale à la quantité minimale qui est nécessaire pour observer un effet significatif, respectivement cosmétique ou pharmaceutique.

Selon une caractéristique particulière de l'invention, l'extrait de Marrubium vulgare est un extrait des parties aériennes, en particulier de tiges et de feuilles.

Selon une autre caractéristique, l'extrait de Marrubium vulgare précité est un extrait organique, en particulier des parties aériennes telles que les tiges et les feuilles, obtenu avantageusement par un procédé comprenant au moins une étape d'extraction avec par exemple un solvant choisi parmi le groupe constitué par l'eau, les alcools comportant de préférence de 1 à 4 atomes de carbone tels que le méthanol, l'éthanol ou le propanol, un mélange hydroalcoolique de ces alcools, les solvants chlorés comportant 1 ou 2 atomes de carbone tels que le chloroforme ou le dichlorométhane, les esters organiques comportant de préférence 3 à 6 atomes de carbone tels que l'acétate d'éthyle. D'une façon plus générale, on peut utiliser également comme solvant, des solvants organiques tels que les hydrocarbures aromatiques, notamment le benzène, le toluène ou le xylène, des hydrocarbures aliphatiques ou aromatiques halogénés, des éthers dialcoyliques, des dialcoylcétones, des alcanols, des acides carboxyliques et leurs esters ou d'autres solvants, comme le diméthylformamide, le dioxanne, le tétrahydrofuranne et le diméthylsulfoxyde. Le rapport de la matière végétale à

2

l'agent d'extraction n'est pas critique, toutefois il est généralement compris entre 1:5 et 1:20 parties en poids, et il est préférentiellement ce 1:10 environ. L'extraction s'effectue à des températures comprises entre la température ambiante et le point d'ébullition du solvant utilisé pour l' extraction. Une technique intéressante d'extraction est la technique dite d'extraction au Soxhlet.

Mais on peut également, de manière simple, effectuer l'extraction au reflux sous pression atmosphérique normale pendant 2 à 4 h, après avoir éventuellement laissé macérer la matière végétale à froid pendant 2 à 4 h dans le solvant d'extraction.

En fin d'extraction, la phase contenant l'extrait est filtrée, puis concentrée et/ou évaporée à sec sous pression réduite ou par lyophilisation.

On obtient ainsi un extrait utilisé dans les compositions selon l'invention.

D'une façon générale, la concentration des extraits utilisés conformément à la présente invention, pour la préparation d'une composition cosmétique ou pharmaceutique, exprimée en poids sec est comprise entre 0,0001 % et 5 % en poids, de préférence entre 0,01 % et 0,5 % en poids, par rapport au poids total de la composition.

Les compositions cosmétiques ou pharmaceutiques, notamment dermatologiques, selon la présente invention, peuvent être préparées sous des formes convenant à divers modes d'administration. En particulier, elles peuvent se présenter sous une forme destinée à l'administration superficielle sur la peau ou le cuir chevelu, telle qu'une crème, un gel, un lait ou une lotion, afin de favoriser la pigmentation de la peau, de prévenir ou ralentir l'apparition des cheveux blancs.

Ainsi, les compositions cosmétiques ou pharmaceutiques, notamment dermatologiques, selon l'invention, trouvent diverses applications en cosmétologie ou en pharmacie notamment en dermatologie, en particulier lorsque la restauration de la pigmentation normale ou son augmentation est recherchée.

Par exemple, ces compositions peuvent être utilisées comme produits solaires pour accélérer ou intensifier le bronzage, ce qui, outre l'avantage esthétique souvent recherché, permet de renforcer les défenses naturelles contre le rayonnement ultraviolet par l'augmentation du taux de mélanine dans l'épiderme. Ces compositions peuvent encore être utilisées, par exemple sous forme de crèmes, pour donner à la peau un aspect plus hâlé, ou encore sous forme de lotions, pour la prévention ou le ralentissement de l'apparition des cheveux blancs. Par ailleurs, en dermatologie, les compositions selon la présente invention peuvent être utilisées comme agents thérapeutiques, seules ou en association avec d'autres médicaments, en particulier en administration topique dans le traitement des dysfonctionnements des mélanocytes.

Avantageusement, les compositions cosmétiques ou pharmaceutiques seton l'invention destinées à l'administration topique, contiennent au moins un agent favorisant la pénétration et la diffusion au niveau des structures cutanées concernées tel que ceux utilisés couramment dans les domaines de la cosmétologie et de la dermo-pharmacie comme par exemple, le glycérol, le propylèneglycol, l'acide oléique ou les huiles essentielles, notamment le menthol et l'eucalyptol.

Selon un mode de réalisation avantageux, une composition cosmétique ou pharmaceutique selon l'invention contient en outre une xanthine, en particulier la 1-méthylxanthine, la 3-méthylxanthine, la 3-isobutylméthylxanthine ou la théophylline, de préférence à une concentration pondérale comprise entre 0,001% et 2%, et encore de préférence comprise entre 0,01% et 0,5%, par rapport au poids total de la composition.

Selon un autre mode de réalisation, en particulier applicable dans le cadre d'une activation de la pigmentation de la peau ou des cheveux, une composition cosmétique ou pharmaceutique selon l'invention contient en outre de la tyrosine ou l'un de ses dérivés tel que le tyrosinate de glucose, la N-malyltyrosine, de préférence à une concentration pondérale comprise entre 0,001% et 10%, par rapport au poids total de la composition.

Selon encore un autre mode de réalisation, en particulier applicable dans le cadre d'une activation de la pigmentation des cheveux, une composition cosmétique ou pharmaceutique selon l'invention contient en outre au moins une autre substance active, à une concentration efficace, choisie parmi les vitamines, en particulier les vitamines B, la quinine ou ses dérivés, les rubéfiants, tels que le nicotinate de méthyle, un surnageant de culture de fibroblastes de papille, tel que défini dans le document EP-A-272 920, des hydrolysats de kératine, des oligoéléments tels que zinc, sélénium, cuivre, des inhibiteurs de 5-α-réductase, tels que progestérone, cyprotérone acétate, minoxidil, acide azélaïque et ses dérivés, 1,4-méthyl-4-azastéroïde, en particulier la 17-β-N, N-diéthylcarbamoyl-4-méthyl-4-aza-5-α-androstan-3-one, ou encore un extrait de Serenoa repens.

Selon encore un autre mode de réalisation, une composition cosmétique ou pharmaceutique selon l'invention contient en outre des phases lamellaires lipidiques hydratées ou des liposomes, incorporant ou non l'extrait de Marrubium vulgare précédemment défini.

Selon un deuxième aspect, la présente invention concerne encore un procédé de fabrication d'une composition cosmétique ou pharmaceutique, notamment dermatologique, destinée en particulier à favoriser la pigmentation de la peau, à prévenir ou ralentir l'apparition des cheveux blancs, à traiter les désordres de la pigmentation de la peau ou des cheveux, caractérisé en ce qu'il comprend l'incorporation d'au moins un extrait de Marrubium vulgare tel que précédemment défini dans un excipient, véhicule ou support cosmétiquement ou pharmaceutiquement acceptable.

Selon un troisième aspect, l'invention concerne l'utilisation d'un extrait de Marrubium vulgare, tel que précédemment défini, pour la préparation d'une composition cosmétique ou pharmaceutique, notamment dermatologique, destinée à favoriser la pigmentation de la peau, à prévenir ou ralentir l'apparition des cheveux blancs ou à traiter des désordres de la pigmentation de la peau ou des cheveux.

Selon encore un autre aspect, la présente invention concerne encore un procédé de traitement cosmétique d'une insuffisance ou de désordres de la pigmentation, caractérisé en ce qu'il comprend l'application d'une quantité cosmétiquement efficace d'au moins un extrait de Marrubium vulgare tel que précédemment défini, avantageusement incorporé dans un excipient, véhicule ou support cosmétiquement acceptable, de préférence sur les zones concernées de la peau ou du cuir chevelu d'un être humain, afin de rétablir une pigmentation normale de la peau ou des cheveux et/ou pour augmenter cette pigmentation.

Dans tous les aspects précédents, l'extrait de Marrubium vulgare, tel que précédemment défini, peut être utilisé en présence de phases lamellaires lipidiques hydratées, ou de liposomes, incorporant ou non ledit extrait. Il est précisé que l'expression "incorporant" couvre le cas où l'extrait est totalement incorporé et celui ou une certaine quantité seulement de cet extrait est incorporée dans les phases lamellaires lipidiques hydratées ou dans les liposomes.

Le terme "lipidique" dans l'expression "phase lamellaire Lipidique" couvre toutes les substances comprenant une chaîne carbonée dite grasse, comportant généralement plus de 5 atomes de carbone, cette substance étant habituellement dénommée "lipide".

Selon l'invention, on utilise à titre de lipide, pour former les phases lamellaires lipidiques, ou les liposomes, des lipides amphiphiles, c'est-à-dire constitués de moléculaires possédant un groupe hydrophile indifféremment ionique ou non ionique et un groupe lipophile, ces lipides amphiphiles étant susceptibles de former des phases lamellaires lipidiques ou des liposomes, en présence d'une phase aqueuse, selon la quantité d'eau dans le mélange.

En particulier, parmi ces lipides, on peut citer : les phospholipides, les phosphoaminolipides, les glycolipides, les alcools gras polyoxyéthylénés, les esters de polyol éventuellement polyoxyéthylénés. De telles substances sont par exemple constituées par une lécithine d'oeuf ou de soja, une phosphatidyle sérine, une sphyngomyéline, un cérébroside ou un stéarate de polyglycérol oxyéthyléné.

De préférence, selon l'invention, on utilise un mélange lipidique constitué d'au moins un lipide amphiphile et d'au moins un lipide hydrophobe tel que stérol, comme le cholestérol ou le $\beta$-sitostérol. La quantité, exprimée en mole, de lipide hydrophobe ne doit généralement pas être supérieure à la quantité de lipide amphiphile, et de préférence elle ne doit pas être supérieure à 0,5 fois cette quantité.

L'incorporation en des phases lamellaires lipidiques hydratées ou dans des liposomes, des composés ou des extraits contenant ces composés, utilisés conformément à la présente invention, peut être réalisée selon des techniques de préparation connues, décrites par exemple dans le document EP-B1-0 087 993 = US-A-4 508 703, éventuellement en combinaison avec le document EP-B1-0 107 559 = US-A-4 621 023.

D'autres buts, caractéristiques et avantages de l'invention apparaîtront clairement à la lumière de la description explicative qui va suivre faite en référence à plusieurs exemples donnés simplement à titre d'illustration.

En effet, la présente invention n'est nullement limitée aux modes de réalisation décrits et illustrés. C'est ainsi, par exemple, qu'elle couvre également une composition cosmétique ou dermatologique destinée à prévenir ou ralentir le blanchiment des sourcils ou des cils.

Dans les exemples, les pourcentages sont donnés en poids, sauf indication contraire.

EXEMPLE 1

Obtention d'un extrait méthanolique de Marrubium vulgare

On traite au reflux pendant 3 h 500 g de parties aériennes de Marrubium vulgare composées de tiges et de feuilles, avec 5 l environ de méthanol.

On filtre les extraits méthanoliques et on évapore le filtrat sous pression réduite, puis on lyophilise. On obtient ainsi l'extrait métranolique de Marrubium vulgaré appelé extrait $I_1$.

EXEMPLE 2

Obtention d'un extrait à l'acétate d'éthyle de Marrubium Vulgare

En opérant comme décrit à l'exemple 1, mais en utilisant la plante entière et comme solvant l'acétate d'éthyle, on obtient un extrait à l'acétate d'éthyle de Marrubium vulgare dénommé extrait $I_2$.

## EXEMPLE 3

### Incorporation d'un extrait de parties aériennes de Marrubium vulgare dans des phases lamellaires lipidiques hydratées ou dans des liposomes

Un extrait de parties aériennes de Marrubium vulgare obtenu conformément à l'exemple 1 est incorporé dans des phases lamellaires lipidiques hydratées ou dans des liposomes selon la technique de préparation suivante :
on pèse :

- lécithine de soja : 1,8 g
- β-sitostérol : 0,2 g
- extrait de Marrubium vulgare lyophilisé $I_1$ de l'exemple 1 : 0,03 g

Ces constituants sont dissous dans un mélange constitué de 25 ml de dichlorométhane et 10 ml de méthanol.

On évapore le mélange ainsi obtenu dans un ballon rotatif à température de 60°C sous pression réduite.

Le film lipidique ainsi obtenu est alors repris et dispersé sous agitation dans de l'eau distillée qsp 50 g, à température ambiante pendant 12 h.

La suspension de vésicules lipidiques obtenue est ensuite homogénéisée par un traitement aux ultrasons pendant 10 min à 4°C, sous puissance de 150 W.

La taille moyenne des liposomes ainsi obtenus est d'environ 140 nm.

Selon une variante de réalisation avantageuse, on gélifie ensuite cette suspension en la mélangeant à 50 g de gel de Carbopol® 940 à 1,25% préparé séparément ce façon classique. On obtient ainsi 100 g environ d'une suspension gélifiée de liposomes encapsulant l'extrait de Marrubium vulgare, dont la concentration est d'environ 0,030% par rapport au poids total de la suspension.

Ce gel est appelé "composition $I_3$" et peut être utilisé tel quel dans le cadre de l'invention.

## EXEMPLE 4

### Mesure de l'efficacité d'un extrait de Marrubium vulgare selon l'invention sur des mélanocytes en culture

### Protocole :

Des mélanocytes murins S91 ensemencés à raison de $2.10^5$ cellules par boîte sont cultivés dans du milieu EMEM complémenté en acides aminés non essentiels et contenant également 1 % de sérum de veau foetal et 0,08 µg/ml de Mitomycine C. 24 h après l'ensemencement, le milieu de culture est remplacé par du milieu frais sans Mitomycine C, comprenant uniquement du milieu EMEM complémenté, 1 % de sérum de veau foetal et, le cas échéant, le produit à tester solubilisé dans du DMSO.

Six jours après ensemencement, on prélève les cellules que l'on isole par centrifugation et on récupère le culot cellulaire que l'on dissout dans de la soude 0,5 N.

On procède à la lecture de la densité optique de la solution obtenue sur un spectrophotomètre à 405 nm, ce qui permet d' évaluer la quantité de mélanine formée par comparaison à la densité optique d'une solution de mélanine de concentration connue.

On procède également au comptage des cellules, et on calcule la quantité de mélanine formée pour $10^6$ cellules.

Les extraits $I_1$ et $I_2$ de Marrubium vulgare ont été testés, à diverses concentrations, en utilisant comme témoin positif une culture n'ayant reçu que de la β-MSH (Melanocyte-Stimulating Hormone), à la concentration de $2.10^{-8}$ M.

L'efficacité E des produits testés sur la stimulation de la mélanogénèse est calculée par la formule suivante :

$$E = \frac{q_p - q_o}{q_t - q_o} \times 100$$

dans laquelle les grandeurs q représentent les quantités de mélanine formée pour $10^6$ cellules :

$q_p$ :      culture recevant le produit à tester
$q_t$ :      culture recevant la βMSH
$q_o$ :      culture témoin ne recevant aucun produit.

L'efficacité E des extraits testés à différentes concentrations, calculée selon la formule ci-dessus, respectivement avec :

a) l'extrait $I_1$ de l'exemple 1 figure au tableau I ci-après, ou

b) avec l'extrait I$_2$ de l'exemple 2 figure au tableau II ci-après.

Les essais ayant été effectués avec trois boîtes par concentration et par produit, les valeurs figurant aux tableaux I et II relatives au nombre de cellules par boîte et à la quantité de mélanine, sont des valeurs moyennes.

L'étude statistique par le test t de Student compare les résultats entre les cultures témoin et les cultures traitées (par les produits selon l'invention ou par la β-MSH).

S = significatif à 5 %,

NS = non significatif à 5 %.

TABLEAU I

| Test avec l'extrait I$_1$ de Marrubium vulgare | | | | |
|---|---|---|---|---|
| Concentration extrait I$_1$ (μg/ml) | nombre de cellules ± écart type par boîte x 10$^{-3}$ | Mélanine μg pour 10$^6$ cellules | Efficacité E | t |
| (Témoin) 0 | 167 ± 0 | 58 ± 4 | 0 | |
| (Témoin positif β-MSH) 0 | 161 ± 5 | 156 ± 11 | 100 | S |
| 1 | 171 ± 7 | 61 ± 1 | + 3 | NS |
| 2,5 | 169 ± 4 | 66 ± 2 | + 8 | NS |
| 10 | 156 ± 6 | 88 ± 5 | +31 | S |

TABLEAU II

| Test avec l'extrait I$_2$ de Marrubium vulgare | | | | |
|---|---|---|---|---|
| Concentration extrait I$_2$ (μg/ml) | nombre de cellules ± écart type par boîte x 10$^{-3}$ | Mélanine μg pour 10$^6$ cellules | Efficacité E | t |
| Témoin | 173 ± 3 | 62 ± 1 | 0 | |
| Témoin β-MSH | 152 ± 13 | 158 ± 10 | 100 | S |
| 1 | 171 ± 7 | 73 ± 4 | +12 | S |
| 2,5 | 166 ± 12 | 80 ± 2 | +19 | S |
| 5 | 157 ± 1 | 87 ± 12 | +27 | S |

Il résulte à partir ces tableaux I et II ci-dessus que les extraits selon l'invention stimulent la production de mélanine dans une proportion significative, ce qui représente un résultat tout à fait inattendu pour un homme de l'art.

On donnera ci-après divers exemples de formulation de compositions cosmétique ou pharmaceutique, notamment dermatologique.

EXEMPLE 5

Lotion favorisant la pigmentation des cheveux :

| | |
|---|---|
| - extrait I$_1$ | 0,15 g |
| - propylèneglycol | 5 g |
| - Crémophor RH40 ® | 1 g |
| - éthanol | 30 g |
| - excipient aqueux parfumé      qsp | 100 g |

Appliquer la lotion sur les cheveux à raison de 1 ml deux fois par jour pendant 6 mois pour l'obtention d'un effet de pigmentation significatif.

EXEMPLE 6

Gel favorisant le bronzage

La préparation du gel est réalisée à partir de deux phases, respectivement A et B, suivantes :

1) Phase A

| | |
|---|---|
| - extrait $I_1$ | 0,1g |
| - malyltyrosine | 2 g |
| - propylèneglycol | 4 g |
| - glycérol | 0,5g |
| - crémophor RH40 ® | 1 g |
| - éthanol | 25 g |
| - excipient aqueux parfumé          qsp | 50 g |

2) Phase B

| | |
|---|---|
| gel de Carbopol 940 ® à 1,25 % | 50 g |

On ajoute progressivement la phase A dans la phase B et on mélange avec un agitateur à hélice jusqu'à dispersion complète.

On obtient ainsi un gel favorisant le bronzage de la peau, qui peut être appliqué avant ou pendant l'exposition solaire.

**Revendications**

1. Composition cosmétique ou pharmaceutique, notamment dermatologique, sous une forme destinée à l'administration superficielle sur la peau ou le cuir chevelu, destinée en particulier à favoriser la pigmentation de la peau, à prévenir ou à ralentir l'apparition des cheveux blancs, caractérisée en ce qu'elle comprend à titre d'ingrédient actif une quantité cosmétiquement ou pharmaceutiquement efficace d'un extrait de Marrubium vulgare, éventuellement dans un excipient cosmétiquement ou pharmaceutiquement acceptable.

2. Composition selon la revendication 1, caractérisée en ce qu'il s'agit d'un extrait des parties aériennes, en particulier de tiges et de feuilles de Marrubium vulgare.

3. Composition selon la revendication 1 ou 2, caractérisée en ce que l'extrait de Marrubium vulgare précité est un extrait organique, en particulier des parties aériennes telles que les tiges et les feuilles, avantageusement obtenu par un procédé comprenant au moins une étape d'extraction avec par exemple un solvant choisi parmi le groupe constitué par l'eau, les alcools comportant de préférence de 1 à 4 atomes de carbone, tels que le méthanol, l'éthanol ou le propanol, un mélange hydroalcoolique de ces alcools, les solvants chlorés comprenant 1 ou 2 atomes de carbone, tels que le chloroforme ou le dichlorométhane, les esters organiques comportant de préférence 3 à 6 atomes de carbone, tels que l'acétate d'éthyle.

4. Composition selon l'une des revendications 1 à 3, caractérisée en ce que la concentration en extrait de Marrubium vulgare précité, exprimée en poids sec, est comprise entre 0,0001 % et 5 % en poids, de préférence entre 0,01 % et 0,5 % en poids, par rapport au poids total de la composition.

5. Composition selon l'une des revendications précédentes, caractérisée en ce que la composition contient en outre une xanthine, en particulier la 1-méthylxanthine, la 3-méthylxanthine, la 3-isobutylméthylxanthine ou la théophylline, de préférence à une concentration pondérale comprise entre 0,001 % et 2 %, et encore de préférence comprise entre 0,01 % et 0,5 %, par rapport au poids total de la composition.

6. Composition selon l'une des revendications précédentes, en particulier pour favoriser la pigmentation de la peau ou des cheveux, caractérisée en ce que la composition contient en outre de la tyrosine ou l'un de ses dérivés tels que le tyrosinate de glucose, la N-malyltyrosine, de préférence à une concentration pondérale comprise entre 0,001 % et 10 %, par rapport au poids total de la composition.

7. Composition selon l'une des revendications 1 à 6, caractérisé en ce qu'elle contient en outre au moins une autre substance active, à une concentration efficace, choisie parmi les vitamines, en particulier les vitamines B, la quinine ou ses dérivés, les rubéfiants, tels que le nicotinate de méthyle, un surnageant de culture de fibroblastes de papille, des hydrolysats de kératine, des oligoéléments tels que zinc, sélénium, cuivre, des inhibiteurs de 5-$\alpha$-réductase, tels que progestérone, cyprotérone acétate, minoxidil, acide azélaïque et ses dérivés, 1,4-méthyl-4-azastéroïde, en particulier la 17-$\beta$-N, N-diéthylcarbamoyl-4-méthyl-4-aza-5-$\alpha$-androstan-3-one, ou encore un extrait de Serenoa repens.

8. Composition selon l'une des revendications précédentes, caractérisée en ce qu'on formule la composition sous une forme destinée à l'administration superficielle sur la peau ou le cuir chevelu, en particulier sous forme de crème, de gel, de lait ou de lotion.

9. Composition selon l'une des revendications précédentes, caractérisée en ce qu'elle contient des phases lamellaires lipidiques hydratées ou des liposomes incorporant ou non l'extrait de Marrubium vulgare précité.

10. Procédé de traitement cosmétique d'une insuffisance ou de désordres de la pigmentation, caractérisé en ce qu'il comprend l'application d'une quantité cosmétiquement efficace d'au moins un extrait de Marrubium vulgare, en particulier tel que précédemment défini à l'une des revendications 2 à 9, avantageusement incorporé dans un excipient, véhicule ou support cosmétiquement acceptable, de préférence sur les zones concernées de la peau ou du cuir chevelu d'un être humain, afin de rétablir une pigmentation normale de la peau ou des cheveux, et/ou pour augmenter cette pigmentation.

11. Utilisation d'un extrait de Marrubium vulgare pour la préparation d'une composition cosmétique ou pharmaceutique, notamment dermatologique, telle que définie à l'une quelconque des revendications 1 à 9, destinée à favoriser la pigmentation de la peau, à prévenir ou ralentir l'apparition des cheveux blancs ou à traiter les désordres de la pigmentation de la peau ou des cheveux.

## Claims

1. Cosmetic or pharmaceutical composition, especially dermatological composition, in a form intended for the surface administration to the skin or scalp, intended in particular for promoting the pigmentation of the skin and preventing or slowing down the appearance of white hair, said composition being characterized in that it comprises, as active ingredient, a cosmetically or pharmaceutically effective amount of an extract of Marrubium vulgare, optionally in a cosmetically or pharmaceutically acceptable excipient.

2. Composition according to claim 1, characterized in that it is an extract of the aerial parts of Marrubium vulgare, in particular the stems and leaves.

3. Composition according to claim 1 or 2, characterized in that the above-mentioned extract of Marrubium vulgare is an organic extract, in particular of the aerial parts such as the stems and leaves, which is advantageously obtained by a process comprising at least one extraction step, for example with a solvent selected from the group consisting of water, alcohols preferably comprising from 1 to 4 carbon atoms, such as methanol, ethanol or propanol, an aqueous-alcoholic mixture of these alcohols, chlorinated solvents comprising 1 or 2 carbon atoms, such as chloroform or dichloromethane, and organic esters preferably comprising from 3 to 6 carbon atoms, such as ethyl acetate.

**4.** Composition according to one of claims 1 to 3, characterized in that the concentration of the above-mentioned extract of Marrubium vulgare, expressed by dry weight, is between 0.0001% and 5% by weight, preferably between 0.01% and 0.5% by weight, based on the total weight of the composition.

**5.** Composition according to one of the preceding claims, characterized in that the composition also contains a xanthine, in particular 1-methylxanthine, 3-methylxanthine, 3-isobutylmethylxanthine or theophylline, preferably at a concentration by weight of between 0.001% and 2% and particularly preferably of between 0.01% and 0.5%, based on the total weight of the composition.

**6.** Composition according to one of the preceding claims, in particular for promoting the pigmentation of the skin or hair, characterized in that the composition also contains tyrosine or a derivative thereof, such as glucose tyrosinate or N-malyltyrosine, preferably at a concentration by weight of between 0.001% and 10%, based on the total weight of the composition.

**7.** Composition according to one of claims 1 to 6, characterized in that it also contains an effective concentration of at least one other active substance selected from vitamins, in particular the B vitamins, quinine or derivatives thereof, rubefacients such as methyl nicotinate, a papilla fibroblast culture supernatant, keratin hydrolyzates, trace elements such as zinc, selenium and copper, 5-$\alpha$-reductase inhibitors such as progesterone, cyproterone acetate and minoxidil, azelaic acid and derivatives thereof, 1,4-methyl-4-azasteroid, in particular 17-$\beta$-N,N-diethylcarharnoyl-4-methyl-4-aza-5-$\alpha$-androstan-3-one, or else an extract of Serenoa repens.

**8.** Composition according to one of the preceding claims, characterized in that the composition is presented in a form intended for suface administration to the skin or scalp, in particular in the form of a cream, a gel, a milk or a lotion.

**9.** Composition according to one of the preceding claims, characterized in that it contains hydrated lipidic lamellar phases or liposomes, which may or may not incorporate the above-mentioned extract of Marrubium vulgare.

**10.** Process for the cosmetic treatment of an insufficiency or of disorders of the pigmentation, characterized in that it comprises the application of a cosmetically effective amount of at least one extract of Marrubium vulgare, in particular as defined above in one of claims 2 to 9, advantageously incorporated into a cosmetically acceptable excipient, vehicle or carrier, preferably on the concerned zones of the skin or scalp of a human, so as to restore normal pigmentation of the skin or hair, and/or increase said pigmentation.

**11.** Use of an extract of Marrubium vulgare for the preparation of a cosmetic or pharmaceutical composition, especially dermatological composition, as defined in any one of claims 1 to 9, intended in particular for promoting the pigmentation of the skin and preventing or slowing down the appearance of white hair or treating pigmentation disorders of the skin or hair.

**Patentansprüche**

**1.** Kosmetische oder pharmazeutische, insbesondere dermatologische Zusammensetzung in einer Form zur oberflächlichen Verabreichung auf die Haut oder die Kopfhaut, insbesondere zur Förderung der Pigmentation der Haut, zur Verhinderung oder Verlangsamung des Auftretens weißer Haare, dadurch gekennzeichnet, daß sie als aktiven Bestandteil eine kosmetisch oder pharmazeutisch wirksame Menge eines Extrakts von Marrubium vulgare, gegebenenfalls in einem kosmetisch oder pharmazeutisch annehmbaren Exzipienten, umfaßt.

**2.** Zusammensetzung nach Anspruch 1, dadurch gekennzeichnet, daß es sich um einen Extrakt von an der Luft befindlichen Teilen, insbesondere den Stielen und den Blättern, von Marrubium vulgare handelt.

**3.** Zusammensetzung nach Anspruch 1 oder 2, dadurch gekennzeichnet, daß der Marrubium vulgare-Extrakt ein organischer Extrakt, insbesondere von an der Luft befindlichen Teilen, wie den Stielen und den Blättern, ist, der vorzugsweise durch ein Verfahren erhalten wird, das zumindest einen Extraktionsschritt beispielsweise mit einem Lösungsmittel, ausgewählt aus der Gruppe bestehend aus Wasser, Alkoholen mit vorzugsweise 1 bis 4 Kohlenstoffatomen, wie Methanol, Ethanol oder Propanol, einer hydroalkoholischen Mischung dieser Alkohole, chlorierten Lösungsmitteln mit 1 oder 2 Kohlenstoffatomen, wie Chloroform oder Dichlormethan, organischen Estern mit vorzugsweise 3 bis 6 Kohlenstoffatomen, wie Ethylacetat, umfaßt.

4. Zusammensetzung nach einem der Ansprüche 1 bis 3, dadurch gekennzeichnet, daß die Konzentration des Marrubium vulgare-Extrakts, ausgedrückt als Trockenmasse, zwischen 0,0001 Masse-% und 5 Masse-%, vorzugsweise zwischen 0,01 Masse-% und 0,5 Masse-%, bezogen auf die Gesamtmasse der Zusammensetzung, beträgt.

5. Zusammensetzung nach einem der vorhergehenden Ansprüche, dadurch gekennzeichnet, daß die Zusammensetzung außerdem ein Xanthin, insbesondere 1-Methylxanthin, 3-Methylxanthin, 3-Isobutylmethylxanthin oder Theophyllin, vorzugsweise in einer Massekonzentration zwischen 0,001 % und 2 %, und auch bevorzugt zwischen 0,01 % und 0,5 %, bezogen auf die Gesamtmasse der Zusammensetzung, enthält.

6. Zusammensetzung nach einem der vorhergehenden Ansprüche, insbesondere zur Förderung der Pigmentation der Haut oder der Haare, dadurch gekennzeichnet, daß die Zusammensetzung außerdem Tyrosin oder eines seiner Derivate, wie Glucosetyrosinat, N-Malyltyrosin, vorzugsweise in einer Massekonzentration zwischen 0,001 % und 10 %, bezogen auf die Gesamtmasse der Zusammensetzung, enthält.

7. Zusammensetzung nach einem der Ansprüche 1 bis 6, dadurch gekennzeichnet, daß sie außerdem zumindest eine andere aktive Substanz in einer wirksamen Konzentration, ausgewählt aus Vitaminen, insbesondere B-Vitaminen, Chinin oder seinen Derivaten, Hautreizmitteln, wie Methylnikotinat, einem Papillenfibroblasten-Kulturüberstand, Keratinhydrolysaten, Spurenelementen, wie Zink, Selen, Kupfer, Inhibitoren von 5-$\alpha$-Reductase, wie Progesteron, Cyproteronacetat, Minoxidil, Azelainsäure und ihren Derivaten, einem 1,4-Methyl-4-azasteroid, insbesondere 17-$\beta$-N,N-Diethylcarbamoyl-4-methyl-4-aza-5-$\alpha$-androstan-3-on, oder auch einen Extrakt von Serenoa repens enthält.

8. Zusammensetzung nach einem der vorhergehenden Ansprüche, dadurch gekennzeichnet, daß die Zusammensetzung in einer Form zur oberflächlichen Verabreichung auf die Haut oder die Kopfhaut, insbesondere in Form einer Creme, eines Gels, einer Milch oder einer Lotion, formuliert wird.

9. Zusammensetzung nach einem der vorhergehenden Ansprüche, dadurch gekennzeichnet, daß sie hydratisierte lamellare Lipidphasen oder Liposomen, die den Marrubium vulgare-Extrakt gegebenenfalls einschließen, enthält.

10. Verfahren zur kosmetischen Behandlung einer Insuffizienz oder von Störungen der Pigmentation, dadurch gekennzeichnet, daß es das Aufbringen einer kosmetisch wirksamen Menge zumindest eines Marrubium vulgare-Extrakts, insbesondere wie vorstehend in einem der Ansprüche 2 bis 9 definiert, der vorteilhaft in einem kosmetisch annehmbaren Exzipienten, Vehikel oder Träger eingeschlossen ist, vorzugsweise auf die betroffenen Zonen der Haut oder der Kopfhaut eines Menschen umfaßt, um eine normale Pigmentation der Haut oder der Haare wiederherzustellen und/oder um diese Pigmentation zu erhöhen.

11. Verwendung eines Marrubium vulgare-Extrakts für die Herstellung einer kosmetischen oder pharmazeutischen, insbesondere dermatologischen Zusammensetzung, wie in einem der Ansprüche 1 bis 9 definiert, zur Förderung der Pigmentation der Haut, zur Verhinderung oder Verlangsamung des Auftretens weißer Haare oder zur Behandlung von Pigmentationsstörungen der Haut oder der Haare.